# EUROPÄISCHE PATENTSCHRIFT

(11) **EP 3 265 045 B1**
(45) Veröffentlichungstag und Bekanntmachung des Hinweises auf die Patenterteilung: **31.10.2018**
(21) Anmeldenummer: 16707129.9
(22) Anmeldetag: 01.03.2016
(51) Int. Cl.: A61J 1/14, A61M 5/178, B65D 47/06, A61J 1/20

(54) **ENTNAHMEVORRICHTUNG FÜR FLÜSSIGKEITEN**
SAMPLING DEVICE FOR LIQUIDS
DISPOSITIF D'EXTRACTION DE LIQUIDES

(30) Priorität: 06.03.2015 EP 15000671
(43) Veröffentlichungstag der Anmeldung: 10.01.2018
(73) Patentinhaber: Coltène/Whaledent GmbH + Co. KG, 89129 Langenau (DE)
(72) Erfinder: MÜLLER, Barbara, 89129 Langenau (DE); SCHLÜTER, Martin, 88239 Wangen (DE); WOLFERT, Johannes, 86462 Langweid (DE); MANNSCHEDEL, Werner, 89129 Langenau (DE)
(74) Vertreter: Forstmeyer, Dietmar
(86) Internationale Anmeldenummer: PCT/EP2016/054322
(87) Internationale Veröffentlichungsnummer: WO 2016/142215

(56) Entgegenhaltungen:
- WO-A1-95/17874
- US-A- 3 853 157
- US-A1- 2010 102 094

## Beschreibung

Die vorliegende Erfindung betrifft eine Entnahmevorrichtung für Flüssigkeiten aus einem Vorratsbehälter, insbesondere im medizinischen Bereich.

Beispielsweise im medizinischen, insbesondere im zahnmedizinischen Bereich spielen Entnahmevorrichtungen für Flüssigkeiten aus einem Vorratsbehälter eine wichtige Rolle, insbesondere für Flüssigkeiten, mit denen Spritzen beschickt oder beladen werden sollen. Die Vorratsbehälter bezeichnet der Fachmann oft kurz als Flasche, Injektionsflasche, Phiole oder Vial. Die Entnahmevorrichtung ermöglicht eine Entnahme von Teilen der in dem Vorratsbehälter gespeicherten Flüssigkeit insbesondere über eine Spritze.

Die DE 10 2013 012 353 A1 beschreibt wie auch die DE 20 2012 007 582 U1 jeweils ein System zur Entnahme medizinischer Flüssigkeiten aus einem Behälter. Die beschriebenen Systeme weisen einen Stopfen auf, der in den Hals des Behälters eingefügt wird, und der in Richtung zum Äußeren des Behälters mittig mit einem weiblichen Luer-Anschluss zur Verbindung mit einer Spritze mit männlichem Luer-Anschluss und zwischen Mitte und Rand des Stopfens mit einer Entlüftungsöffnung versehen ist.

Gemäß der Lehre der DE 20 2012 007 582 U1 ist der weibliche Luer-Anschluss ein Luer-Lock-Anschluss, und der Stopfen ist gegenüber dem weiblichen Luer-Lock-Anschluss in Richtung zum Inneren des Behälters mit einem über eine Öffnung in dem Stopfen mit dem weiblichen Luer-Lock-Anschluss in Verbindung stehenden Saugschlauch versehen.

Gemäß der Lehre der DE 10 2013 012 353 A1 ist der weibliche Luer-Anschluss ein in den Stopfen derart vertieft eingearbeiteter Luer-Lock-Anschluss, dass die Oberfläche des Luer-Lock-Anschlusses mit der Oberfläche des Stopfens bündig abschließt, und der Stopfen gegenüber dem weiblichen Luer-Lock-Anschluss in Richtung zum Inneren des Behälters mit einem über eine Durchgangsöffnung in dem Stopfen mit dem weiblichen Luer-Lock-Anschluss in Verbindung stehenden Saugschlauch mit einer Durchgangsöffnung versehen ist, deren Öffnungsweite gleich der Öffnungsweite der Durchgangsöffnung in dem Stopfen ist.

Die US 8 671 994 B2 beschreibt eine Vorrichtung zur Befüllung von Injektionsspritzen mit einem Fluidkanal, der einen am unteren Ende eines Vorratsgefäßes befindlichen Auslass mit einem Anschluss für Injektionsspritzen verbindet. Eine weitere Vorrichtung zur Entnahme einer Flüssigkeit aus einem Vorratsbehälter ist bekannt aus US 2010/102094 A1.

Diese und weitere bekannte Lösungen sind mit verschiedenen Nachteilen verbunden. Einige Lösungen erfordern, dass die Flasche auf dem Kopf steht oder zur Entnahme auf den Kopf gestellt werden muss, wobei die Gefahr besteht, dass Flüssigkeit in unerwünschter Weise aus der Flasche ausläuft. Bei einigen bekannten Lösungen ist keine wirklich einhändige Entnahme der Flüssigkeit möglich, bei der der Arzt bzw. Zahnarzt oder seine Helferin wirklich nur eine Hand benötigt, um eine Spritze anzusetzen und so aus der Flasche Flüssigkeit zu entnehmen, ohne dass Flüssigkeit durch unerwünschtes Auslaufen verloren geht oder in die Flasche zurückläuft, was die Gefahr einer Verunreinigung der in der Flasche gespeicherten Flüssigkeit mit sich bringt.

Bei einigen bekannten Lösungen müssen Ventile von Hand betätigt werden und/oder die Flasche oder eine Anordnung mehrerer Flaschen (d.h. eine sogenannte Station oder Beladungsstation) muss von Hand festgehalten werden. Bei diesen oder anderen bekannten Lösungen erhöht eine große Zahl an Kleinteilen oder Bedienelementen die Gefahr eines Bedienfehlers und erschwert die Reinigung der Anordnung.

Sofern ein insbesondere freiliegender Luer-Lock-Anschluss für Injektionsspritzen vorhanden ist, sind in diesem Anschluss meist relativ große Löcher vorhanden. Dadurch besteht Auslaufgefahr. Nach einer Benutzung muss deshalb immer ein Deckel aufgeschraubt werden. Weiterhin besteht die Gefahr, dass durch die Öffnungen Schmutz in die Flasche gelangt.

Aufgrund der komplexen und komplizierten aus mehreren Schritten bestehenden Inbetriebnahme werden bekannte Stationen häufig fehlerhaft in Betrieb genommen. Beim Nachfüllen der Flasche kann es außerdem leicht zum Austreten oder Verschütten der Flüssigkeit kommen. Nach der Inbetriebnahme fließt die Flüssigkeit bei diesen Anordnungen in einen Vorratsbehälter der Station, weshalb in der Station, die insbesondere Schläuche und Verbindungen aufweist, immer Flüssigkeit steht. Die Gefahr des Flüssigkeitsaustritts ist hier sehr hoch, und die Erfahrung zeigt, dass viele Stationen nach einer gewissen Zeit undicht werden.

Da der Kunststoff der Einzelkomponenten der Station über längere Zeiträume in Kontakt mit der Flüssigkeit bleibt, besteht eine erhöhte Gefahr, dass die jeweils verwendete, chemisch häufig aggressive Flüssigkeit den Kunststoff bis zum Totalversagen angreift. Einzelteile solcher Flaschen oder Stationen können meist nicht ohne aufwendige Reparatur ersetzt werden.

Das Reservoir solcher Stationen lässt sich häufig nicht oder nur mit hohem Aufwand reinigen. Deshalb können sich hier leicht Verschmutzungen anreichern.

Aufgabe der vorliegenden Erfindung ist es, eine neuartige, gegenüber dem Stand der Technik nach Möglichkeit verbesserte Vorrichtung zur Entnahme von Flüssigkeiten aus einem Vorratsbehälter anzugeben. Insbesondere ist es auch eine Aufgabe der Erfindung für die zahnmedizinische Technik eine Vorrichtung zur Entnahme von Flüssigkeiten, insbesondere von Spüllösungen, beispielsweise einer Natriumhypochloridlösung zum Spülen von Wurzelkanalhöhlen, anzugeben.

Diese Aufgabe wird durch einen Gegenstand nach dem unabhängigen Patentanspruch gelöst. Vorteilhafte Weiterbildungen der Erfindung ergeben sich aus den abhängigen Ansprüchen. Dabei können Merkmale oder Merkmalsgruppen einiger Ausführungsformen mit Merkmalen oder Merkmalsgruppen anderer Ausführungsformen durch den Fachmann auch zu weiteren, hier nicht ausdrücklich beschriebenen, ebenfalls vorteilhaften Ausführungsformen dieser Erfindung kombiniert werden.

Erfindungsgemäß wird eine Vorrichtung zur Entnahme einer Flüssigkeit aus einem Vorratsbehälter geschaffen, die einen Körper aufweist, der so ausgestaltet ist, dass er mit einer Öffnung des Vorratsbehälters verbunden werden kann. An einem oberen Ende des Körpers ist ein Deckel mit einem Rand angebracht. Seitlich an den Rand des Deckels angrenzend ist ein Anschluss zur Verbindung der Vorrichtung mit einer Spritze angeordnet. Eine an ihrem unteren Ende senkrecht in den Vorratsbehälter führende Saugleitung, mündet an ihrem oberen Ende in den Anschluss.

Eine solche Vorrichtung ist für eine einhändige Bedienung besonders geeignet. Sie ist sehr einfach und intuitiv in Betrieb zu nehmen.

Der Körper der Vorrichtung ist vorzugsweise so ausgestaltet, dass er lösbar, besonders vorzugsweise kraftschlüssig, mit einer Öffnung des Vorratsbehälters verbunden werden kann. Gemäß einer bevorzugten Ausführungsform kann der Körper an der Vorrichtung arretiert werden. Dadurch kann gewährleistet werden, dass die Vorrichtung immer optimal in der Halterung ausgerichtet ist.

In einer bevorzugten Ausführungsform der Erfindung ist der Körper wenigstens bereichsweise hohl. In seinem Inneren weist er eine sich zu ihrem unteren Ende hin, vorzugsweise stetig und/oder gestuft, verengende, insbesondere wenigstens bereichsweise konische Struktur auf, durch welche die Saugleitung in den Vorratsbehälter führt. Vorzugsweise ist der Körper so ausgeführt, dass ein Abnehmen des Deckels nicht möglich ist; so kann der Deckel z.B. durch Kleben an dem Körper befestigt werden.

Die Saugleitung ist vorzugsweise als Saugschlauch ausgeführt.

Vorzugsweise schließt der Saugschlauch oder die Saugleitung das untere Ende der konischen Struktur luftdicht und/oder flüssigkeitsdicht ab, besonders vorzugsweise, indem der äußere Durchmesser des Saugschlauches genau in den inneren Durchmesser der konischen Struktur an deren unterem Ende passt. Einem unbeabsichtigten Verschütten oder einer Verunreinigung der Flüssigkeit wird auf diese Weise entgegengewirkt.

In einer weiteren bevorzugten Ausführungsform der Erfindung ist der Körper so ausgestaltet, dass er lösbar mit der Öffnung des Vorratsbehälters verbunden und vorzugsweise von oben auf diese Öffnung aufgesetzt oder mit Hilfe eines im Innern des Körpers angebrachten Gewindes auf diese Öffnung aufgeschraubt werden kann. Dadurch kann der Körper, z.B. bei Verschmutzung, leicht ausgetauscht werden.

In einer weiteren bevorzugten Ausführungsform der Erfindung weist der Körper in seinem Inneren wenigstens einen Hohlraum auf, der so ausgestaltet ist, dass Luft durch diesen Hohlraum in den Vorratsbehälter oder aus dem Vorratsbehälter wenigstens dann strömen kann, wenn eine Druckdifferenz zwischen dem Inneren des Vorratsbehälters und dem Außenraum besteht. Der Körper kann auch zwei oder mehrere solche Hohlräume aufweisen. Derartige Ausgestaltungen der erfindungsgemäßen Vorrichtung fördern die Belüftung des Vorratsbehälters und wirken der Ausbildung einer unerwünschten Druckdifferenz entgegen, die dazu führen könnte, dass Flüssigkeit in unerwünschter Weise durch den Saugschlauch oder durch die Saugleitung in den oder aus dem Vorratsbehälter fließt. Gemäß einer bevorzugten Ausführungsform sind die Hohlräume so gestaltet, dass keine großen Mengen an Flüssigkeit ausgeschüttet werden können.

In einer weiteren bevorzugten Ausführungsform der Erfindung ist der Anschluss als vorzugsweise weiblicher Luer-Anschluss, besonders vorzugsweise als Luer-Lock-Anschluss, zur Verbindung mit einer Spritze mit vorzugsweise männlichem Luer-Anschluss oder Luer-Lock-Anschluss ausgestaltet. Derartige Ausgestaltungen der erfindungsgemäßen Vorrichtung ermöglichen eine sichere Verbindung handelsüblicher und branchenüblicher Injektionsspritzen und/oder Schlauchverbindungen mit dem Anschluss der erfindungsgemäßen Vorrichtung. In alternativer Weise ist der Anschluss als Ventil, z.B. als ein Luer-Lock Ventil ausgestaltet. Dadurch befindet sich in der Saugleitung nach erstmaligem Aufziehen der Spritze keine Luft mehr, solange noch - auch nur eine geringe Menge an - Flüssigkeit im Vorratsbehälter vorhanden ist. Weitere Spritzen können so befüllt werden ohne eine größere Menge Luft in die Spritze zu ziehen.

In einer weiteren bevorzugten Ausführungsform der Erfindung weist der Körper der Vorrichtung an der dem Anschluss gegenüberliegenden Seite einen Vorsprung auf. Der Vorsprung ist vorzugsweise derart ausgestaltet, dass er in eine Aussparung einer Halterung eingreifen und/oder einrasten kann. Derartige Ausgestaltungen der erfindungsgemäßen Vorrichtung erleichtern deren Integration in eine Halterung, insbesondere in eine Tischhalterung oder eine Wandhalterung, wodurch eine gewisse Standfestigkeit der Vorrichtung erreicht werden kann, die einer leichten und sicheren einhändigen Bedienung förderlich ist. Außerdem kann so ein unbeabsichtigtes Lösen der Vorrichtung und/oder des Vorratsbehälters aus der Halterung erschwert werden.

In einer weiteren bevorzugten Ausführungsform der Erfindung weist der Deckel eine Öffnung zur Belüftung oder Entlüftung der Vorrichtung auf. Auf diese Weise kann eine ausreichende Belüftung der Vorrichtung und des Vorratsbehälters auch bei geschlossenem Deckel gewährleistet werden. Optional kann in der Öffnung oder an einer anderen Stelle zwischen Öffnung und Vorratsbehälter ein Filter vorgesehen werden, um eine Verunreinigung der Flüssigkeit im Vorratsbehälter durch verunreinigte einströmende Luft etc. zu verhindern. Gemäß einer weiteren Ausführungsform ist der Körper nicht vollständig luftdicht, so dass keine spezielle Belüftungsöffnung notwendig ist.

Erfindungsgemäß wird ferner eine Beladungsstation geschaffen, die einen Vorratsbehälter, eine Halterung und eine Entnahmevorrichtung gemäß einer der Ausführungsformen der Erfindung aufweist. Die Halterung ist vorzugsweise als Tischhalterung und besonders vorzugsweise mit einer Grundplatte ausgeführt, auf der vorzugsweise eine vertikale oder senkrecht stehende Struktur mit einer Ausnehmung angeordnet ist, in welche der Vorsprung der erfindungsgemäßen Entnahmevorrichtung eingreifen und/oder einrasten kann.

In einer bevorzugten Ausführungsform der erfindungsgemäßen Beladungsstation ist der Vorsprung an der dem Anschluss gegenüberliegenden Seite des Körpers derart ausgestaltet oder angeordnet, dass er in eine Ausnehmung in der Halterung derart eingreift oder einrastet, dass ein unbeabsichtigtes Lösen der Vorrichtung aus der Halterung erschwert wird oder praktisch unmöglich ist.

Ferner wird eine Verwendung eines Erzeugnisses nach einer der beschriebenen Ausführungsformen der Erfindung zur Entnahme einer Spülflüssigkeit zur Spülung eines Wurzelkanals vorgesehen. Die Merkmale der erfindungsgemäßen Entnahmevorrichtung und ihrer bevorzugten Ausführungsformen tragen in besonderer Weise dazu bei, dass die speziellen Anforderungen an die Einfachheit der Bedienung, insbesondere der möglichst einhändigen Flüssigkeitsentnahme aber auch der Reinigung der Vorrichtung, und an die Einhaltung von Hygienevorschriften, wie sie für eine zahnärztliche Praxis typisch sind, erfüllt werden können.

Die Erfindung wird nachstehend anhand bevorzugter Ausführungsbeispiele und mit Bezug auf die beigefügten Figuren näher erläutert.
Fig. 1 zeigt einen schematischen Querschnitt durch ein Ausführungsbeispiel einer erfindungsgemäßen Vorrichtung zur Entnahme von Flüssigkeiten aus einem Vorratsbehälter;
Fig. 2 zeigt eine schematische, perspektivische Ansicht eines Ausführungsbeispiels einer erfindungsgemäßen Vorrichtung zur Entnahme von Flüssigkeiten aus einem Vorratsbehälter;
Fig. 3 zeigt eine schematische, perspektivische Ansicht eines Ausführungsbeispiels einer Beladungsstation mit Ausführungsbeispielen eines Vorratsbehälters und einer erfindungsgemäßen Vorrichtung zur Entnahme von Flüssigkeiten aus einem Vorratsbehälter.

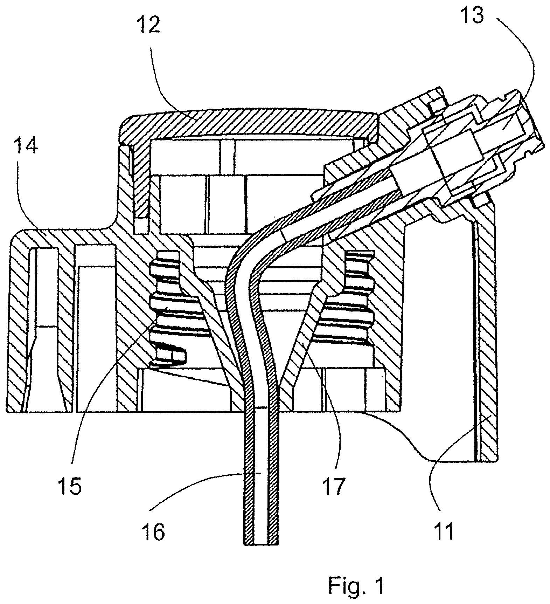

Figur 1 zeigt einen schematischen Querschnitt durch ein Ausführungsbeispiel einer erfindungsgemäßen Vorrichtung zur Entnahme von Flüssigkeiten aus einem (in der Figur 1 nicht gezeigten) Vorratsbehälter. Die Vorrichtung weist einen Körper 11 auf, der so ausgestaltet ist, dass er mit einer Öffnung des Vorratsbehälters verbunden werden kann, einen am oberen Ende des Körpers 11 angebrachten Deckel 12 mit einem Rand, einen an den Rand des Deckels 12 seitlich angrenzend angeordneten Anschluss 13 zur Verbindung der Vorrichtung mit einer Spritze und eine an ihrem unteren Ende senkrecht in den Vorratsbehälter führende Saugleitung 16, die an ihrem oberen Ende in den Anschluss 13 mündet. Der Körper 11 ist wenigstens teilweise hohl, und in seinem Inneren weist er eine sich zu ihrem unteren Ende hin verengende, wenigstens bereichsweise konische Struktur 17 auf, durch welche die Saugleitung 16 in den Vorratsbehälter führt. Der Körper 11 der Vorrichtung weist an der dem Anschluss 13 gegenüberliegenden Seite einen Vorsprung 14 auf.

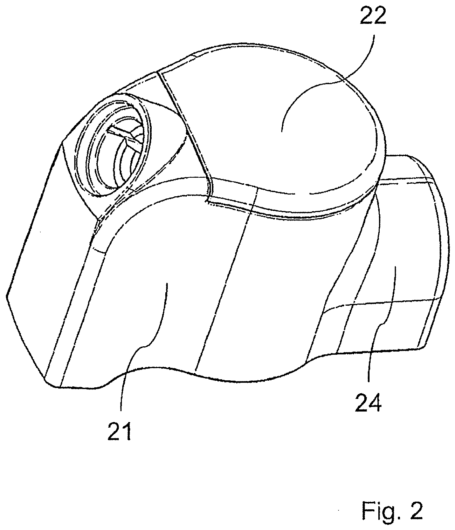

Figur 2 zeigt eine schematische, perspektivische Ansicht eines Ausführungsbeispiels einer erfindungsgemäßen Vorrichtung zur Entnahme von Flüssigkeiten aus einem (in der Figur 2 nicht gezeigten) Vorratsbehälter. Die Vorrichtung weist einen Körper 21, der so ausgestaltet ist, dass er mit einer Öffnung des Vorratsbehälters verbunden werden kann, einen am oberen Ende des Körpers 21 angebrachten Deckel 22 mit einem Rand, und eine an den Rand des Deckels 22 seitlich angrenzend angeordnete Einrichtung für einen Anschluss (nicht dargestellt) zur Verbindung der Vorrichtung mit einer Spritze auf. Der Körper 21 der Vorrichtung weist an der der Einrichtung für einen Anschluss gegenüber liegenden Seite einen Vorsprung 24 auf.

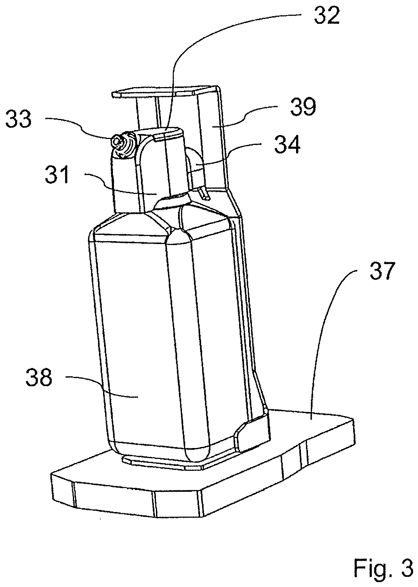

Figur 3 zeigt eine schematische, perspektivische Ansicht eines Ausführungsbeispiels einer Beladungsstation mit Ausführungsbeispielen eines Vorratsbehälters 38 und einer erfindungsgemäßen Vorrichtung 31, 32, 33, 34 zur Entnahme von Flüssigkeiten aus diesem Vorratsbehälter 38. Der Vorratsbehälter 38 steht auf einer Grundplatte 37 einer Halterung 37, 39, die eine vertikale oder senkrecht stehende Struktur 39 aufweist. Der Vorsprung 34 der Entnahmevorrichtung greift in eine Ausnehmung der vertikalen Struktur 39 ein oder ist in diese Ausnehmung eingerastet. In Figur 3 ist wegen der gewählten Perspektive nicht dargestellt, dass die Struktur 39 zusätzlich oder alternativ einen Vorsprung aufweisen kann, der in eine Ausnehmung eingreift, die der Ausnehmung 18 in Figur 1 entspricht.

In den Figuren wurden folgende Bezugszeichen verwendet:
- 11: Entnahmevorrichtung
- 12: Deckel
- 13: Anschluss
- 14: Vorsprung
- 15: Gewinde
- 16: Saugleitung, Saugschlauch
- 17: konische Struktur
- 18: Ausnehmung
- 21: Entnahmevorrichtung
- 22: Deckel
- 24: Vorsprung
- 31: Entnahmevorrichtung
- 32: Deckel
- 33: Anschluss
- 34: Vorsprung
- 37: Grundplatte
- 38: Vorratsbehälter
- 39: Halterung

## Patentansprüche

1. Vorrichtung zur Entnahme einer Flüssigkeit aus einem Vorratsbehälter, wobei diese Vorrichtung aufweist:
a) einen Körper (11, 21, 31), der so ausgestaltet ist, dass er mit einer Öffnung des Vorratsbehälters verbunden werden kann; und
b) eine an ihrem unteren Ende senkrecht in den Vorratsbehälter führende Saugleitung (16), die an ihrem oberen Ende in einen Anschluss (13, 33) mündet; **dadurch gekennzeichnet, dass** die Vorrichtung weiterhin aufweist:
c) einen am oberen Ende des Körpers (11, 21, 31) angebrachten Deckel (12, 22, 32) mit einem Rand; und
d) den an den Rand des Deckels (12, 22, 32) seitlich angrenzend angeordneten Anschluss (13, 33) zur Verbindung der Vorrichtung mit einer Spritze.

2. Entnahmevorrichtung nach Anspruch 1, **dadurch gekennzeichnet, dass** der Körper wenigstens teilweise hohl ist und in seinem Inneren eine sich zu ihrem unteren Ende hin verengende, insbesondere wenigstens bereichsweise konische, Struktur (17) aufweist, durch welche die Saugleitung (16) in den Vorratsbehälter führt.

3. Entnahmevorrichtung nach einem der vorhergehenden Ansprüche, **dadurch gekennzeichnet, dass** der Körper (11, 21, 31) so ausgestaltet ist, dass er lösbar mit der Öffnung des Vorratsbehälters verbunden, vorzugsweise von oben auf diese Öffnung aufgesetzt oder mit Hilfe eines im Innern des Körpers angebrachten Gewindes (15) auf diese Öffnung aufgeschraubt und/oder gegebenenfalls am Vorratsbehälter arretiert werden kann.

4. Entnahmevorrichtung nach einem der vorhergehenden Ansprüche, **dadurch gekennzeichnet, dass** der Körper (11, 21, 31) in seinem Inneren wenigstens einen Hohlraum (19) aufweist, der so ausgestaltet ist, dass Luft durch diesen Hohlraum in den Vorratsbehälter oder aus dem Vorratsbehälter wenigstens dann strömen kann, wenn eine Druckdifferenz zwischen dem Inneren des Vorratsbehälters und dem Außenraum besteht.

5. Entnahmevorrichtung nach einem der vorhergehenden Ansprüche, **dadurch gekennzeichnet, dass** der Anschluss (13, 33) als vorzugsweise weiblicher Luer-Anschluss, besonders vorzugsweise als weiblicher Luer-Lock-Anschluss, zur Verbindung mit einer Spritze mit vorzugsweise männlichem Luer-Anschluss, besonders vorzugsweise mit einem männlichem Luer-Lock-Anschluss, ausgestaltet ist.

6. Entnahmevorrichtung nach einem der Ansprüche 1 bis 4, **dadurch gekennzeichnet, dass** der Anschluss (13, 33) als Ventil, vorzugsweise weibliches Luer-Ventil, besonders vorzugsweise als weibliches Luer-Lock-Ventil, zur Verbindung mit einer Spritze mit vorzugsweise männlichem Luer-Anschluss, besonders vorzugsweise mit einem männlichem Luer-Lock-Anschluss, ausgestaltet ist.

7. Entnahmevorrichtung nach einem der vorhergehenden Ansprüche, **dadurch gekennzeichnet, dass** der Körper (11, 21, 31) der Vorrichtung an der dem Anschluss (13, 33) gegenüberliegenden Seite einen Vorsprung (14, 24, 34) aufweist.

8. Entnahmevorrichtung nach einem der vorhergehenden Ansprüche, **dadurch gekennzeichnet, dass** sie eine Öffnung zur Belüftung oder Entlüftung der Vorrichtung aufweist.

9. Beladungsstation, aufweisend einen Vorratsbehälter (38), eine Halterung (39) und eine Entnahmevorrichtung (31, 32, 33, 34) nach einem der vorhergehenden Ansprüche.

10. Beladungsstation nach Anspruch 9, **dadurch gekennzeichnet, dass** der Vorsprung (14, 24, 34) an der dem Anschluss (13, 33) gegenüberliegenden Seite des Körpers (11, 21, 31) in eine Ausnehmung in der Halterung (39) derart eingreift oder einrastet, dass ein unbeabsichtigtes Lösen der Vorrichtung aus der Halterung erschwert wird oder praktisch unmöglich ist.

## Claims

1. A device for withdrawal of a fluid from a storage container, the device having:
a) a body (11, 21, 31) which is configured so that it can be connected to an opening of the storage container;
b) a suction line (16) which at its lower end leads vertically into the storage container and at its upper end opens into a connector (13, 33);
**characterized in that** the device further comprises:
c) a rimmed cover (12, 22, 32) mounted on the upper end of the body (11, 21, 31); and
d) a connector (13, 33) arranged laterally adjacent to the rim of the cover (12, 22, 32) for connecting the device to a syringe.

2. A withdrawal device according to claim 1, **characterized in that** the body is at least partly hollow and has in its interior a structure (17) which tapers towards its lower end and is especially at least in some regions conical, through which structure the suction line (16) leads into the storage container.

3. A withdrawal device according to one of the preceding claims, **characterized in that** the body (11, 21, 31) is configured so that it can be releasably connected to the opening of the storage container, preferably placed onto that opening from above or screwed onto that opening with the aid of a thread (15) provided in the interior of the body and/or optionally locked in place on the storage container.

4. A withdrawal device according to one of the preceding claims, **characterized in that** the body (11, 21, 31) has in its interior at least one cavity (19) which is configured so that air is able to flow through that cavity into or out of the storage container at least when there is a pressure difference between the interior of the storage container and the exterior space.

5. A withdrawal device according to one of the preceding claims, **characterized in that** the connector (13, 33) is preferably configured as a female Luer connector, especially preferred as a female Luer lock connector, for connection to a syringe preferably having a male Luer connector, especially preferred a male Luer lock connector.

6. A withdrawal device according to one of claims 1 to 4, **characterized in that** the connector (13, 33) is configured as a valve, preferably as a female Luer valve, especially preferred as a female Luer lock valve, for connection to a syringe, preferably having a male Luer connector, especially preferred having a male Luer lock connector.

7. A withdrawal device according to one of the preceding claims, **characterized in that** the body (11, 21, 31) of the device has a projection (14, 24, 34) on its side opposite the connector (13, 33).

8. A withdrawal device according to one of the preceding claims, **characterized in that** it comprises an opening for admitting air into or discharging air from the device.

9. A loading station comprising a storage container (38), a holder (39) and a withdrawal device (31, 32, 33, 34) according to one of the preceding claims.

10. A loading station according to claim 9, **characterized in that** the projection (14, 24, 34) on the side of the body (11, 21, 31) opposite the connector (13, 33) engages in or snaps into a recess in the holder (39) in such a way that unintentional release of the device from the holder is made more difficult or basically impossible.

## Revendications

1. Dispositif de prélèvement d'un liquide d'un réservoir de stockage, dans lequel ce dispositif comprend :
a) un corps (11, 21, 31), qui est conçu de manière à pouvoir être relié à une ouverture du réservoir de stockage ; et
b) une conduite d'aspiration (16) qui mène verticalement par son extrémité inférieure dans le réservoir de stockage et qui débouche par son extrémité supérieure dans un raccord (13, 33) ;
**caractérisé en ce que** le dispositif présente en outre :
c) un couvercle (12, 22, 32) monté à l'extrémité supérieure du corps (11, 21, 31) et pourvu d'un bord ; et
d) le raccord (13, 33) agencé de manière latéralement adjacente au bord du couvercle (12, 22, 32) et destiné à relier le dispositif à une seringue.

2. Dispositif de prélèvement selon la revendication 1, **caractérisé en ce que** le corps est au moins en partie creux et présente en son sein une structure (17), s'amincissant en direction de son extrémité inférieure, en particulier conique au moins par endroits, à travers laquelle la conduite d'aspiration (16) mène dans le réservoir de stockage.

3. Dispositif de prélèvement selon l'une quelconque des revendications précédentes, **caractérisé en ce que** le corps (11, 21, 31) est conçu de manière à pouvoir être relié de manière détachable à l'ouverture du réservoir de stockage, de préférence posé par le haut sur cette ouverture ou vissé sur cette ouverture à l'aide d'un filet (15) appliqué à l'intérieur du corps et/ou le cas échéant bloqué sur le réservoir de stockage.

4. Dispositif de prélèvement selon l'une quelconque des revendications précédentes, **caractérisé en ce que** le corps (11, 21, 31) présente en son sein au moins une cavité (19), qui est conçue de sorte que de l'air peut entrer dans le réservoir de stockage ou sortir du réservoir de stockage en passant par cette cavité, au moins lorsqu'il existe une différence de pression entre l'intérieur du réservoir de stockage et l'espace extérieur.

5. Dispositif de prélèvement selon l'une quelconque des revendications précédentes, **caractérisé en ce que** le raccord (13, 33) est réalisé de préférence sous la forme d'un raccord Luer femelle, de manière particulièrement préférée sous la forme d'un raccord Luer-Lock femelle, pour la liaison à une seringue présentant de préférence un raccord Luer mâle, de manière particulièrement préférée un raccord Luer-Lock mâle.

6. Dispositif de prélèvement selon l'une quelconque des revendications 1 à 4, **caractérisé en ce que** le raccord (13, 33) est réalisé sous la forme d'une soupape, de préférence une soupape Luer femelle, de manière particulièrement préférée sous la forme d'une soupape Luer-Lock femelle, pour la liaison à une seringue présentant de préférence un raccord Luer mâle, de manière particulièrement préférée un raccord Luer-Lock mâle.

7. Dispositif de prélèvement selon l'une quelconque des revendications précédentes, **caractérisé en ce que** le corps (11, 21, 31) du dispositif présente une partie saillante (14, 24, 34) sur la face opposée au raccord (13, 33).

8. Dispositif de prélèvement selon l'une quelconque des revendications précédentes, **caractérisé en ce qu'**il présente une ouverture pour l'aération ou la désaération du dispositif.

9. Station de chargement, comprenant un réservoir de stockage (38), un support (39) et un dispositif de prélèvement (31, 32, 33, 34) selon l'une quelconque des revendications précédentes.

10. Station de chargement selon la revendication 9, **caractérisée en ce que** la partie saillante (14, 24, 34) s'insère ou s'encliquète sur la face du corps (11, 21, 31) opposée au raccord (13, 33) dans un évidement dans le support (39), de telle manière qu'un détachement accidentel du dispositif du support est rendu plus difficile ou quasiment impossible.
